(19) Europäisches Patentamt
European Patent Office
Office européen des brevets

(11) EP 1 009 366 B1

# (12) FASCICULE DE BREVET EUROPEEN

(45) Date de publication et mention de la délivrance du brevet:
**26.03.2003 Bulletin 2003/13**

(21) Numéro de dépôt: **98942778.6**

(22) Date de dépôt: **24.08.1998**

(51) Int Cl.[7]: **A61K 7/06**, A61K 7/48

(86) Numéro de dépôt international:
**PCT/FR98/01845**

(87) Numéro de publication internationale:
**WO 99/009939 (04.03.1999 Gazette 1999/09)**

(54) **COMPOSITIONS COSMETIQUES CONTENANT UN COPOLYMERE BLOC SILICONE POLYOXYALKYLENE AMINE ET UN AGENT CONDITIONNEUR ET LEURS UTILISATIONS**

KOSMETISCHE ZUSAMMENSETZUNGEN ENTHALTEND EIN POLYOXYALKYLIERTES AMINIERTES SILIKON BLOCKMISCHPOLYMER UND EIN KONDITIONIERUNGSMITTEL UND DEREN VERWENDUNGEN

COSMETIC COMPOSITIONS COMPRISING A POLYOXYALKYLATED AMINATED SILICONE BLOCK POLYMER AND A CONDITIONING AGENT AND THEIR USE

(84) Etats contractants désignés:
**AT BE CH CY DE DK ES FI FR GB GR IE IT LI LU MC NL PT SE**

(30) Priorité: **25.08.1997 FR 9710617**

(43) Date de publication de la demande:
**21.06.2000 Bulletin 2000/25**

(73) Titulaire: **L'OREAL**
**75008 Paris (FR)**

(72) Inventeurs:
- **RESTLE, Serge**
  **F-95390 Saint-Prix (FR)**
- **CAUWET-MARTIN, Danièle**
  **F-75011 Paris (FR)**

(74) Mandataire:
**Le Blainvaux Bellegarde, Françoise et al**
**L'OREAL-DPI**
**6 rue Bertrand Sincholle**
**92585 Clichy Cedex (FR)**

(56) Documents cités:
**EP-A- 0 492 657**
**EP-A- 0 643 961**
**EP-A- 0 684 041**
**FR-A- 2 709 954**
**FR-A- 2 709 955**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 009 366 B1

## Description

La présente invention concerne de nouvelles compositions cosmétiques comprenant dans un milieu cosmétiquement acceptable au moins un agent conditionneur choisis parmi les poly-α-oléfines, les huiles fluorées, les cires fluorées, les gommes fluorées, les esters d'acides carboxyliques ayant au moins 10 atomes de carbone, les polymères cationiques, les silicones insolubles dans le milieu, les huiles minérales, végétales ou animales et au moins une silicone aminée polyoxyalkylénée de type $(AB)_n$, A étant un bloc polysiloxane et B étant un bloc polyoxyalkyléné comportant au moins un groupement amine.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légèreté du cheveu), un manque de lissage (cheveu non homogène de la racine à la pointe) et une insuffisance concernant la brillance.

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

On a également préconisé pour améliorer les propriétés conditionnantes des produits capillaires (FR-A-2709954 et FR-A-2709955), d'utiliser des compositions cosmétiques pour le traitement des cheveux contenant l'association d'un polymère cationique ou amphotère et d'une silicone polyoxyalkylénée non ionique de type (AB)n, A étant un bloc polysiloxane et B étant un bloc polyoxyalkyléné. Ces compositions ne donnent cependant pas encore entière satisfaction en ce qui concerne les propriétés de lissage et de douceur conférées aux cheveux. De plus, les cheveux sont alourdis après des applications répétées.

En résumé, il s'avère que les compositions cosmétiques actuelles contenant des agents conditionneurs, ne donnent pas complètement satisfaction.

La demanderesse a maintenant découvert que l'association d'une silicone aminée particulière avec certains agents conditionneurs permet de remédier à ces inconvénients du fait d'un effet de synergie.

Ainsi, à la suite d'importantes recherches menées sur la question, il a maintenant été trouvé par la Demanderesse qu'en introduisant une silicone aminée polyoxyalkylénée de type (AB)n , A étant un bloc polysiloxane et B étant un bloc polyoxyalkyténé comportant au moins un groupement amine dans les compositions en particulier capillaires de l'art antérieur à base d'agents conditionneurs, il est possible de limiter, voire supprimer, les problèmes généralement liés à l'emploi de telles compositions, à savoir en particulier l'alourdissement, le manque de lissage et de douceur, des cheveux, tout en conservant les autres propriétés cosmétiques avantageuses qui sont attachés aux compositions à base d'agents conditionneur.

Cette association apporte des propriétés cosmétiques nettement améliorées par rapport aux propriétés obtenues avec l'un ou l'autre des constituants utilisé seul.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins une silicone aminée polyoxyalkylénée de type (AB)n , A étant un bloc polysiloxane et B étant un bloc polyoxyalkyléné comportant au moins un groupement amine et au moins un agent conditionneur choisi parmi les poly-α-oléfines, les huiles fluorées, les cires fluorées, les gommes fluorées, les esters d'acides carboxyliques ayant au moins 10 atomes de carbone, les polymères cationiques, les silicones insolubles dans le milieu, les huiles minérales, végétales ou animales.

Un autre objet de l'invention concerne un procédé de traitement des matières kératiniques, telles que les cheveux, caractérisé en ce qu'il consiste à appliquer sur lesdites matières des compositions cosmétiques selon l'invention.

Les différents objets de l'invention vont maintenant être détaillés. L'ensemble des significations et définitions des composés utilisés dans la présente invention données ci-dessous sont valables pour l'ensemble des objets de l'invention.

Les silicones aminées polyoxyalkylénées de type (AB)n , A étant un bloc polysiloxane et B étant un bloc polyoxyalkyléné comportant au moins un groupement amine peuvent être constituées d'unités répétitives de formule générale suivante :

$$[SiMe_2\text{-O- }(SiMe_2O)_xSiMe_2\text{ - R-N(H)-R'-O -}(C_2H_4O)_a\text{ - }(C_3H_6O)_b\text{ - R'-N(H)-R-}]$$

dans laquelle :

- a est un nombre entier supérieur ou égal à 1, de préférence compris entre 5 et 200 et encore plus particulièrement entre 5 et 100.
- b est un nombre entier compris entre 0 et 200, de préférence compris entre 4 et 200 et encore plus particulièrement entre 5 et 100.
- R, identiques ou différents, représentent un groupe organique divalent qui est lié à l'atome de silicium adjacent par une liaison carbone-silicium et à un atome d'azote,
- R', identiques ou différents, représentent un groupe organique divalent qui est lié à l'atome d'oxygène adjacent par une liaison carbone-oxygène et à un atome d'azote,

R est de préférence un radical hydrocarboné en $C_2$-$C_{12}$ comportant éventuellement un ou plusieurs hétéroatomes tels que l'oxygène. Plus particulièrement, R désigne un radical éthylène, propylène linéaire ou ramifié, butylène linéaire ou ramifié ou $-CH_2CH_2CH_2OCH(OH)CH_2-$.
R' est un radical hydrocarboné en $C_2$-$C_{12}$ comportant éventuellement un ou plusieurs hétéroatomes tels que l'oxygène. Plus particulièrement R' désigne un radical alkylène divalent comme par exemple l'éthylène, le propylène linéaire ou ramifié ou le butylène linéaire ou ramifié.

Les blocs siloxane représentent généralement entre 50 et 95% en moles par rapport au poids total de la silicone et plus particulièrement compris entre 70 et 85% en moles.

Le taux d'amine est généralement compris entre 0,02 et 0,5 meq/g de copolymère dans une solution à 30% dans le dipropylèneglycol et plus particulièrement entre 0,05 et 0,2.

Le poids moléculaire moyen en poids de la silicone est de préférence compris entre 5000 et 1000000 et encore plus particulièrement entre 10000 et 200000.

La préparation des copolymères blocs mis en oeuvre dans le cadre de la présente invention se fait par des procédés connus de l'homme du métier, par exemple par réaction de silicone $\alpha$-$\bar{\omega}$-diépoxyde ou dichloro avec un polyoxyalkylène $\alpha$-$\bar{\omega}$-diamine.

La silicone aminée polyoxyalkylénée est utilisée de préférence en une quantité comprise entre 0,01 et 20% en poids du poids total de la composition. Plus préférentiellement, cette quantité est comprise entre 0,1 et 15% en poids et encore plus particulièrement entre 0,5 et 10 % en poids.

Les agents de conditionnement peuvent se présenter sous forme liquide, semi-solide ou solide tels que par exemple des huiles, des cires ou des gommes.

Selon l'invention, les agents conditionneurs doivent être choisis parmi les poly-$\alpha$-oléfines, les huiles fluorées, les cires fluorées, les gommes fluorées et les esters d'acides carboxyliques ayant au moins 10 atomes de carbone, les polymères cationiques, les silicones insolubles dans le milieu, les huiles minérales, végétales ou animales et leurs mélanges. Les agents conditionneurs préférés selon l'invention sont les poly-$\alpha$-oléfines, les polymères cationiques et les silicones insolubles dans le milieu.

Les poly-$\alpha$-oléfines sont en particulier :

- de type polybutène, hydrogéné ou non, et de préférence polyisobutène, hydrogéné ou non.
  On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
  A titre d'exemples de poly-$\alpha$-oléfines utilisables dans le cadre de la présente invention, on peut plus particulièrement mentionner les produits vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),

- de type polydécène, hydrogéné ou non.
  De tels produits sont vendus par exemple sous les dénominations ETHYLFLO par la société ETHYL CORP., et d'ARLAMOL PAO par la société ICI.

Les esters d'acides carboxyliques sont de préférence insolubles dans l'eau à une concentration de 0,1% en poids à 25°C. Le nombre total de carbone des esters est supérieur ou égal à 10 et de préférence inférieure à 100 et plus particulièrement inférieur à 80. De préférence, ils ne possèdent pas de propriétés tensioactives.

Les esters d'acides peuvent être mono, di, tri ou tétracarboxyliques.

Les esters d'acides liquides à une température inférieure ou égale à 30 °C sont les plus particulièrement

préférés.

Les esters d'acides monocarboxyliques sont notamment les monoesters d'acides aliphatiques saturés ou insaturés, linéaires ou ramifiés en $C_1$-$C_{26}$ et d'alcools aliphatiques saturés ou insaturés, linéaires ou ramifiés en $C_1$-$C_{26}$ , le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en C12-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le myristate d'isopropyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; le myristate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.
On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle

Les huiles fluorées, les cires fluorées, les gommes fluorées sont par exemple les perfluoropolyéthers décrits notamment dans la demande de brevet EP-A-486135 et les composés fluorohydrocarbonées décrites notamment dans la demande de brevet WO 93/11103. L'enseignement de ces deux demandes est totalement inclus dans la présente demande à titre de référence.

Le terme de composés fluorohydrocarbonées désigne des composés dont la structure chimique comporte un squelette carboné dont certains atomes d'hydrogène ont été substitués par des atomes de fluor.

Les huile fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Les huiles pouvant être utilisées dans les compositions de l'invention sont choisies préférentiellement dans le groupe formé par :

- les huiles animales ou végétales formées par des esters d'acide gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule $R_9COOR_{10}$ dans laquelle $R_9$ représente le reste d'un acide gras supérieur comportant de 7 à 29 atomes de carbone et $R_{10}$ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- des hydrocarbures, tels que l'hexadécane et l'huile de paraffine ;
- des esters d'acide minéral et d'un alcool ;

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337

354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) Les copolymères vinylpyrrolidone-acrylate ou -méthacrylate de dialkylamino-alkyle quaternisés ou non, tels que les produits vendus sous la dénomination "Gafquat" par la Société ISP, comme par exemple Gafquat 734, 755 ou HS100 ou bien le produit dénommé "Copolymère 937". Ces polymères sont décrits en détail dans les brevets français 2 077 143 et 2 393 573.

(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les produits commercialisés répondant à cette définition sont plus particulièrement les produits vendus sous la dénomination "Celquat L 200" et "Celquat H 100" par la Société National Starch.

(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.

De tels produits sont commercialisés notamment sous les dénominations commerciales de JAGUAR C13 S, JAGUAR C 15, JAGUAR C 17 ou JAGUAR C162 par la société MEYHALL.

(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4

atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(9) les cyctopolymères de méthyl diallyl amine ou de diméthyl diallyl ammonium tels que les homopolymères ou copolymères comportant comme constituant principal de la chaîne des motifs répondant aux formules (VI) ou (VI') :

(VI) 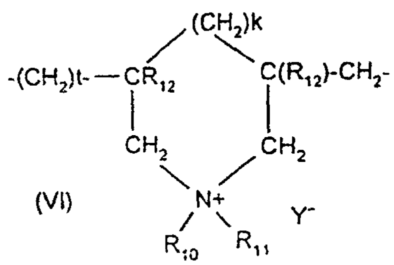

(VI') 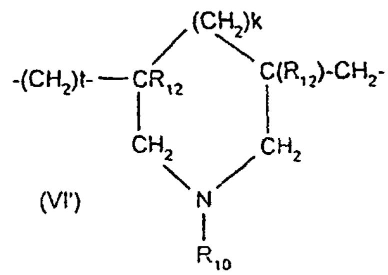

formules dans lesquelles k et t sont égaux à 0 ou 1, la somme k + t étant égale à 1 : $R_{12}$ désigne un atome d'hydrogène ou un radical méthyle ; $R_{10}$ et $R_{11}$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou $R_{10}$ et $R_{11}$ peuvent désigner conjointement avec l'atome d'azote auquel ils sont rattachés, des groupement hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

Parmi les polymères définis ci-dessus, on peut citer plus particulièrement l'homopolymère de chlorure de diméthyldiallylammonium vendu sous la dénomination "Merquat 100" par la société Merck et ses homologues de faibles masses moléculaires moyenne en poids.

(10) le polymère de diammonium quaternaire contenant des motifs récurants répondant à la formule :

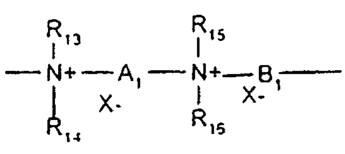 (VII)

formule (VII) dans laquelle :

R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont

rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;

A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et

$X^-$ désigne un anion dérivé d'un acide minéral ou organique;

A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-

dans lequel D désigne :

a) un reste de glycol de formule : -O-Z-O-, où Z désigne un radical hydrocarboné linéaire ou ramifié ou un groupement répondant à l'une des formules suivantes :

-(CH2-CH2-O)x-CH2-CH2-

-[CH2-CH(CH3)-O]y-CH2-CH(CH3)-

où x et y désignent un nombre entier de 1 à 4, représentant un degré de polymérisation défini et unique ou un nombre quelconque de 1 à 4 représentant un degré de polymérisation moyen ;

b) un reste de diamine bis-secondaire tel qu'un dérivé de pipérazine ;

c) un reste de diamine bis-primaire de formule : -NH-Y-NH-, où Y désigne un radical hydrocarboné linéaire ou ramifié, ou bien le radical bivalent

-CH2-CH2-S-S-CH2-CH2- ;

d) un groupement uréylène de formule : -NH-CO-NH- ;

De préférence, $X^-$ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

Selon l'invention, on peut utiliser plus particulièrement les polymères choisis parmi le composés de formule (VII) dans laquelle R13, R14, R15 et R16 représentent le radical méthyle, A1 représente le radical de formule -(CH2)3- et B1 représente le radical de formule -(CH2)6- et $X^-$ représente l'anion chlorure et le composé de formule (VII) dans laquelle R13 et R14 représentent le radical éthyle, R15 et R16 représentent le radical méthyle, A1 et B1 représentent le radical de formule -(CH2)3- et $X^-$ représente l'anion bromure.

(11) les polymères de polyammonium quaternaires constitués de motifs de formule (VIII):

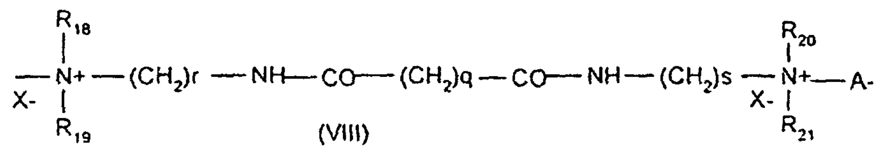

(VIII)

formule dans laquelle :

R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,

où p est égal à 0 ou à un nombre entier compris entre 1 et 6, sous réserve que R18, R19, R20 et R21 ne représentent pas simultanément un atome d'hydrogène,

r et s, identiques ou différents, sont des nombres entiers compris entre 1 et 6,

q est égal à 0 ou à un nombre entier compris entre 1 et 34,

X désigne un atome d'halogène,

A désigne un radical d'un dihalogénure ou représente de préférence -CH2-CH2-O-CH2-CH2-.

De tels composés sont notamment décrits dans la demande de brevet EP-A-122 324.

On peut par exemple citer parmi ceux-ci, les produits "Mirapol A 15", "Mirapol AD1", "Mirapol AZ1" et "Mirapol 175" vendus par la société Miranol.

(12) les homopolymères ou copolymères dérivés des acides acrylique ou méthacrylique et comportant des motifs :

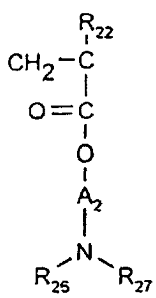

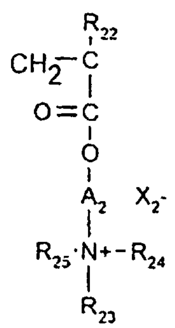

et/ou

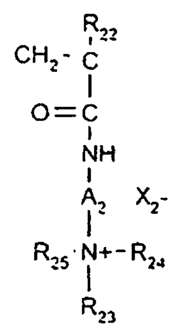

dans lesquels les groupements R22 désignent indépendamment H ou CH3,

les groupements A1 désignent indépendamment un groupe alcoyle linéaire ou ramifié de 1 à 6 atomes de carbone ou un groupe hydroxyalcoyle de 1 à 4 atomes de carbone,

les groupements R23, R24, R25, identiques ou différents, désignant indépendamment un groupe alcoyle de 1 à 18 atomes de carbone ouun radical benzyle,

les groupements R26 et R27 représentent unatome d'hydrogène ou un groupement alcoyle de 1 à 6 atomes de carbone,

$X_2$ désigne un anion, par exemple méthosulfate ou halogénure, tel que chlorure ou bromure.

Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.

(13) Les polymères quaternaires de vinylpyrrolidone et de vinylimidazole tels que par exemple les produits commercialisés sous les dénominations Luviquat FC 905, FC 550 et FC 370 par la société B.A.S.F..

(14) Les polyamines comme le Polyquart H vendu par HENKEL, référencé sous le nom de « POLYETHYLENEGLYCOL (15) TALLOW POLYAMINE » dans le dictionnaire CTFA.

(15) Les polymères réticulés de chlorure de méthacryloyloxyéthyltriméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale. Cette dispersion est commercialisée sous le nom de « SALCARE SC 95 » par la Société ALLIED COLLOIDS.

D'autres polymères cationiques utilisables dans le cadre de l'invention sont des polyalkylèneimines, en particulier des polyétnylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères, en particulier les polymères ou copolymères de chlorure de diméthyldiallylammonium et d'acrylamide, vendus sous les dénominations « MERQUAT 100 », « MERQUAT 550 » et « MERQUAT S » par la Société MERCK, les polysaccharides cationiques et plus particulièrement la gomme de guar modifiée par du chlorure de 2,3-époxypropyl triméthylammonium vendue sous la dénomination « JAGUAR C13S » par la Société MEYHALL.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :

(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.

On peut également citer les cyclocopolymères du type diméthylsiloxanes/méthylakylsiloxane, tel que la "SILICONE VOLATILE FZ 3109" commercialisée par la société UNION CARBIDE, de structure chimique :

avec D :

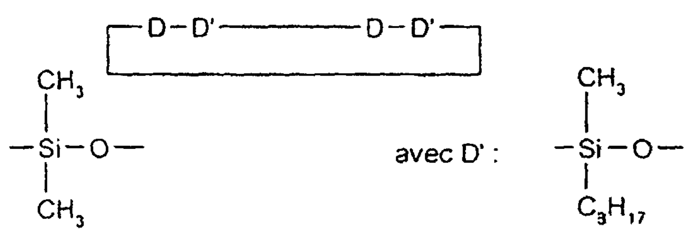

avec D' :

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;

(ii) les silicones volatiles linéaires ayant 2 à 9 atomes de silicium et possédant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25°C et de préférence $1.10^{-5}$ à 1 $m^2/s$.

Parmi ces polyalkylsiloxanes, on peut citer à titre non limitatif les produits commerciaux suivants :

- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl ($C_1$-$C_{20}$) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de $1.10^{-5}$ à $5.10^{-2} m^2/s$ à 25°C.

Parmi ces polyalkylarylsiloxanes on peut citer à titre d'exemple les produits commercialisés sous les dénominations suivantes :

. les huiles SILBIONE de la série 70 641 de RHONE POULENC ;
. les huiles des séries RHODORSIL 70 633 et 763 de RHONE POULENC ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :

- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;

. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;

. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6} m^2/$

s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :

$R_2SiO_{2/2}$, $R_3SiO_{1/2}$, $RSiO_{3/2}$ et $SiO_{4/2}$ dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en $C_1$-$C_4$, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl ($C_{12}$) méthicone copolyol commercialisée par la société DOW CORNING sous la dénomination Q2 5200 ;

- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;

- des groupements thiols comme les produits commercialisés sous les dénominations "GP 72 A" et "GP 71" de GENESEE ;

- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;

- des groupements hydroxylés comme les polyorganosiloxanes à fonction hydroxyalkyle décrits dans la demande de brevet français FR-A-85 16334 répondant à la formule (V) :

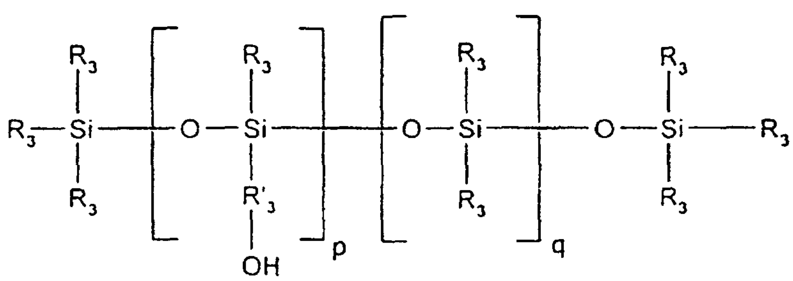

(V)

dans laquelle les radicaux $R_3$ identiques ou différents sont choisis parmi les radicaux méthyle et phényle ; au moins 60 % en mole des radicaux $R_3$ désignant méthyle ; le radical $R'_3$ est un chaînon alkylène divalent hydrocarboné en $C_2$-$C_{18}$ ; p est compris entre 1 et 30 inclus ; q est compris entre 1 et 150 inclus ;

- des groupements acyloxyalkyle tels que par exemple les polyorganosiloxanes décrits dans le brevet US-A-4957732 et répondant à la formule (VI) :

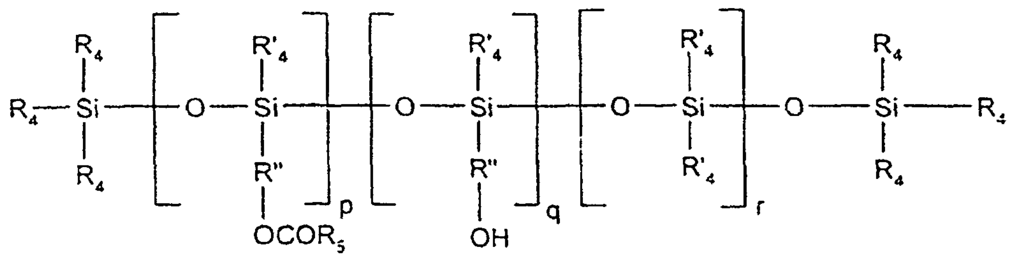 (VI)

dans laquelle

$R_4$ désigne un groupement méthyle, phényle, $-OCOR_5$, hydroxyle, un seul des radicaux $R_4$ par atome de silicium pouvant être OH ;

$R'_4$ désigne méthyle, phényle ; au moins 60 % en proportion molaire de l'ensemble des radicaux $R_4$ et $R'_4$ désignant méthyle ;

$R_5$ désigne alkyle ou alcényle en $C_8$-$C_{20}$ ;

R" désigne un radical alkylène hydrocarboné divalent, linéaire ou ramifié en $C_2$-$C_{18}$ ;

r est compris entre 1 et 120 inclus ;

p est compris entre 1 et 30 ;

q est égal à 0 ou est inférieur à 0,5 p, p + q étant compris entre 1 et 30 ; les polyorganosiloxanes de formule (VI) peuvent contenir des groupements :

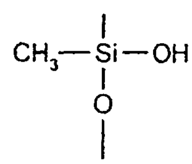

dans des proportions ne dépassant pas 15 % de la somme p + q + r.

- des groupements anioniques du type carboxylique comme par exemple dans les produits décrits dans le brevet EP 186 507 de la société CHISSO CORPORATION, ou de type aikylcarboxyliques comme ceux présents dans le produit X-22-3701E de la société SHIN-ETSU ; 2-hydroxyalkylsulfonate; 2-hydroxyalkylthiosulfate tels que les produits commercialisés par la société GOLDSCHMIDT sous les dénominations "ABIL. S201" et "ABIL S255".

- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :

a) 50 à 90% en poids d'acrylate de tertiobutyle ;
b) 0 à 40% en poids d'acide acrylique ;
c) 5 à 40% en poids de macromère siliconé de formule :

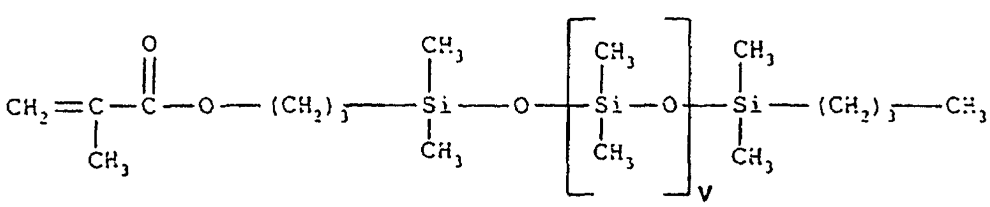

avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont :

- les silicones non volatiles choisies dans la famille des polyalkylsiloxanes à groupements terminaux triméthylsilyle telles que les huiles ayant une viscosité comprise entre 0,2 et 2,5 $m^2/s$ à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;

- les mélanges d'organopolysiloxanes et de silicones cycliques tels que le produit Q2 1401 commercialisé par la société DOW CORNING, et le produit SF 1214 commercialisé par la société GENERAL ELECTRIC ;

- les mélanges de deux PDMS de viscosités différentes notamment d'une gomme et d'une huile tels que le produit SF 1236 commercialisé par la société GENERAL ELECTRIC ;

- la résine d'organopolysiloxane commercialisée sous la dénomination DOW CORNING 593 ;

- les polysiloxane à groupements aminés tels que les amodiméthicone ou les triméthylsilylamodiméthicone ;

Il est bien entendu possible de mettre en oeuvre des mélanges d'agents conditionneur.

Selon l'invention, le ou les agents conditionneurs peuvent représenter de 0,001 % à 10 % en poids, de préférence de 0,005 % à 7 % en poids, et encore plus préférentiellement de 0,01 % à 5 % en poids, du poids total de la composition finale.

Les compositions de l'invention contiennent en outre avantageusement au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

(i) <u>Tensioactif(s) anionique(s)</u>:

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulronates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensais d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les aminés grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particuliérement bien dans le cadre de la présente invention.

(iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits commercialisés sous les dénomination MIRANOL, tels que décrits dans les brevets US-2 528 378 et US-2 781 354 et de structures :

$$R_2\text{ -CONHCH}_2\text{CH}_2\text{ -N}(R_3)(R_4)(\text{CH}_2\text{COO-}) \qquad (2)$$

dans laquelle : $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;
et

$$R_5\text{-CONHCH}_2\text{CH}_2\text{-N(B)(C)} \qquad (3)$$

dans laquelle :

B représente $-CH_2CH_2OX'$, C représente $-(CH_2)_2$ -Y', avec z = 1 ou 2,
X' désigne le groupement $-CH_2CH_2$-COOH ou un atome d'hydrogène
Y' désigne -COOH ou le radical $-CH_2$ - CHOH - SO3H
$R_5$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélange avec :

- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

La composition de l'invention peut également contenir au moins un additif choisi parmi les épaississants, les silicones solubles, les tensioactifs, les parfums, les agents nacrants, les conservateurs, les filtres solaires siliconés ou non, les protéines, les vitamines, les céramides, les pseudocéramides, les cires et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques et cationiques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 2 % à 50 % en poids, de préférence de 10 % à 35 % en poids, et encore plus préférentiellement de 12 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.
Dans tout ce qui suit ou ce qui précède, les pourcentages exprimés sont en poids.
L'invention va être maintenant plus complètement illustrée à l'aide des exemples suivants qui ne sauraient être considérés comme la limitant aux modes de réalisation décrits.
Dans les exemples, MA signifie matière active.

## EXEMPLE 1

On a réalisé trois compositions de shampooing, l'une conforme à l'invention (composition A) et les autres comparatives (compositions B et C) :

| | A<br>Invention | B<br>Comparatif | C<br>Comparatif |
|---|---|---|---|
| - Lauryléthersulfate de sodium ($C_{12}$/$C_{14}$ à 70/30) à 2,2 moles d'oxyde d'éthylène | 17 gMA | 17 gMA | 17 gMA |
| - Cocobétaïne à 32% MA (DEHYTON AB 30 de HENKEL) | 3 gMA | 3 gMA | 3 gMA |
| - Silicone aminée polyoxyalkylénée de type (AB)n (SILSOFT A843 de OSI) | 1,5 g MA | 2 g | - |
| - Gomme de guar cationique (JAGUAR C13S de MEYHALL) | 0,5 g | - | 2 g |
| - Distéarate d'éthylèneglycol | 2,5 g | 2,5 g | 2,5 g |
| - Monoisopropanolamide d'acides de coprah | 2 g | 2 g | 2 g |
| -NaOH qs pH | 8,5 | 8,5 | 8,5 |
| - Eau déminéralisée qs | 100 g | 100 g | 100 g |
| (MA = matière active) | | | |

On effectue un shampooing en appliquant environ 1 g de la composition A sur des mèches de 2,7 g de cheveux décolorés préalablement mouillés. On fait mousser le shampooing, on laisse pauser pendant 15 minutes puis on rince abondamment à l'eau.
On procède selon le même mode opératoire que ci-dessus avec les compositions comparatives B et C.
Un panel d'experts a évalué le lissage des cheveux séchés.
Le test utilisé a pour objet le classement, par un jury, de chaque série de 3 échantillons en fonction croissante ou décroissante de l'efficacité du démêlage. Les 3 mèches de la même série sont présentées simultanément au juge. On lui demande de les classer de la plus lisse à la moins lisses. L'analyse statistique des résultats est effectuée à l'aide des tables de A. KRAMER (Food Technology 17 - (12), 124 - 125 1963).

**RESULTATS:**

| Testeurs | 1 | 2 | 3 | 4 | 5 | 6 | 7 | 8 | 9 | 10 | Σ des rangs |
|---|---|---|---|---|---|---|---|---|---|---|---|
| Mèche A | 1 | 1 | 1 | 1 | 1 | 1 | 3 | 1 | 1 | 1 | 12 |
| Mèche B | 3 | 2 | 2 | 3 | 3 | 3 | 1 | 2 | 3 | 2 | 24 |
| Mèche C | 2 | 3 | 3 | 2 | 2 | 2 | 2 | 3 | 2 | 3 | 24 |

**CONCLUSION :**

Pour la composition A selon l'invention, les résultats obtenus avec les mèches traitées par les compositions contenant le mélange de la silicone aminée de type (AB)n et du polymère cationique sont significativement supérieurs (au seuil de 5 %) à ceux obtenus avec les deux autres compositions contenant chacun des composés utilisé seul.

**EXEMPLE 2**

On a réalisé trois compositions de shampooing, l'une conforme à l'invention (composition A) et les autres comparatives (compositions B et C) :

| | A<br>Invention | B<br>Comparatif | C<br>Comparatif |
|---|---|---|---|
| - Lauryléthersulfate de sodium ($C_{12}$/$C_{14}$ à 70/30) à 2,2 moles d'oxyde d'éthylène | 17 gMA | 17 gMA | 17 gMA |
| - Cocobétaïne à 32% MA (DEHYTON AB 30 de HENKEL) | 3 gMA | 3 gMA | 3 gMA |
| - Silicone aminée polyoxyalkylénée de type (AB)n (SILSOFT A843 de OSI) | 1,5 g MA | 2 g | - |
| - Polyisobutène hydrogéné (2,2,4,4,6,6,8-heptaméthylnonane) (ARLAMOL HD par la société ICI) | 0,5 g | - | 2 g |
| - Distéarate d'éthylèneglycol | 2,5 g | 2,5 g | 2,5 g |
| - Monoisopropanolamide d'acides de coprah | 2 g | 2 g | 2 g |
| - NaOH qs pH | 8,5 | 8,5 | 8,5 |
| - Eau déminéralisée qs | 100 g | 100 g | 100 g |
| (MA = matière active) | | | |

On effectue un shampooing en appliquant environ 1 g de la composition A sur des mèches de 2,7 g de cheveux décolorés préalablement mouillés. On fait mousser le shampooing, on laisse pauser pendant 15 minutes puis on rince abondamment à l'eau.
On procède selon le même mode opératoire que ci-dessus avec les compositions comparatives B et C.
Un panel d'experts a évalué le lissage des cheveux mouillés.
Le test utilisé a pour objet le classement, par un jury, de chaque série de 3 échantillons en fonction croissante ou décroissante de l'efficacité du démêlage. Les 3 mèches de la même série sont présentées simultanément au juge. On lui demande de les classer de la plus lisse à la moins lisses. L'analyse statistique des résultats est effectuée à l'aide des tables de A. KRAMER (Food Technology 17 - (12), 124 - 125 1963).

**RESULTATS :**

| Testeurs | 1 | 2 | 3 | 4 | 5 | 6 | 7 | 8 | 9 | 10 | Σ des rangs |
|---|---|---|---|---|---|---|---|---|---|---|---|
| Mèche A | 1 | 1 | 1 | 1 | 1 | 1 | 1 | 1 | 1 | 1 | 10 |
| Mèche B | 2 | 3 | 2 | 3 | 3 | 3 | 3 | 3 | 2 | 3 | 27 |
| Mèche C | 3 | 2 | 3 | 2 | 2 | 2 | 2 | 2 | 3 | 2 | 23 |

**CONCLUSION :**

Pour la composition A selon l'invention, les résultats obtenus avec les mèches traitées par les compositions contenant le mélange de la silicone aminée de type (AB)n et de la poly-$\alpha$-oléfine sont significativement supérieurs (au seuil de 5 %) à ceux obtenus avec les deux autres compositions contenant chacun des composés utilisé seul.

**EXEMPLE 3**

On réalise une composition de shampooing conforme à l'invention

| | |
|---|---|
| - Lauryléthersulfate d'ammonium à 2,2 moles d'oxyde d'éthylène | 14 gMA |

(suite)

| | |
|---|---|
| - Cocoamphodiacétate de sodium<br>(MIRANOL C2M CONC de RHODIA CHIMIE) | 2,5 gMA |
| - Silicone aminée polyoxyalkylénée de type (AB)n<br>(SILSOFT A843 de OSI) | 1,5 g MA |
| - Palmitate d'isopropyle | 1 g |
| - Distéarate d'éthytèneglycol | 2,5 g |
| - Monoisopropanolamide d'acides de coprah | 2 g |
| - HCl qs pH | 7,5 |
| - Eau déminéralisée qs | 100 g |
| (MA = matière active) | |

**EXEMPLE 4**

On réalise une composition de shampooing conforme à l'invention

| | |
|---|---|
| - Lauryléther carboxylate de sodium AKYPO RLM 45 de CHEM Y | 15 gMA |
| - Alkylpolyglycoside (KAG 40 de KAO) | 3 gMA |
| - Silicone aminée polyoxyalkylénée de type (AB)n (SILSOFT A843 de OSI) | 2 g MA |
| - Stéarate d'isopropyle | 2 g |
| - Parfum, conservateur | qs |
| - HCl qs pH | 7 |
| - Eau déminéralisée qs | 100 g |
| (MA = matière active) | |

**EXEMPLE 5**

On prépare un après-shampooing de composition suivante :

| | |
|---|---|
| Hydroxyéthylcellulose vendu sous le nom de NATROSOL 250 HHR par AQUALON | 1g |
| Mélange alcool cétylstéarylique/alcool cétylstéarylique 30 OE | 3g |
| Polydiméthylsiloxane (DC200-60000cst de DOW CORNING) | 3g |
| SILSOFT A843 | 2g |
| Conservateur, parfum qs | |
| Eau q.s.p. | 100g |

**EXEMPLE 6**

On prépare une composition oxydante de déformation permanente ou de défrisage des cheveux de composition suivante :

| | |
|---|---|
| Mélange alcool cétylstéarylique/alcool cétylstéarylique 30 OE | 4 g |
| Monoéthanolamide d'acide alkyléther carboxylique à 2 moles d'oxyde d'éthylène (AMINOL A 15 de CHEM Y | 1 g |

(suite)

| | |
|---|---|
| SILSOFT A843 | 1gMA |
| Copolymère de chlorure de méthacrylate de triméthyl ammonium et d'acrylamide (SALCARE SC92 de ALLIED COLLOID) | 0,5 gMA |
| Peroxyde d'hydrogène | 2,5 g |
| Acide diéthylène triamine pentaacétique, sel pentasodique | 0,05 g |
| Stabilisant | 0,06 g |
| Acide phosphorique | 0,17 g |
| Eau déminéralisée q.s.p. | 100g |

**EXEMPLE 6**

On prépare une composition oxydante de déformation permanente ou de défrisage des cheveux de composition suivante :

| | |
|---|---|
| Alcool cétylique | 2 g |
| Alcool stéarylique à 2,2 moles d'oxyde d'éthylène | 0,5 g |
| SILSOFT A843 | 1gMA |
| Phosphate acide d'alcool cétéarylique / alcool cétéarylique | 3,2 g |
| Huile de vaseline | 3 g |
| Peroxyde d'hydrogène | 2,5 g |
| Acide diéthylène triamine pentaacétique, sel pentasodique | 0,15 g |
| Stabilisant | 0,06 g |
| Acide phosphorique | 0,17 g |
| Eau déminéralisée q.s.p. | 100g |

**Revendications**

1. Composition cosmétique, **caractérisée par le fait qu'**elle comprend, dans un milieu cosmétiquement acceptable, au moins une silicone aminée polyoxyalkyténée de type (AB)n , A étant un bloc polysiloxane et B étant un bloc polyoxyalkyléné comportant au moins un groupement amine et au moins un agent conditionneur choisi parmi les poly-$\alpha$-oléfines, les huiles fluorées, les cires fluorées, les gommes fluorées, les esters d'acides carboxyliques ayant au moins 10 atomes de carbone, les polymères cationiques, les silicones insolubles dans le milieu, les huiles minérales, végétales ou animales.

2. Composition selon la revendication 1, **caractérisée par** le fait la silicone aminée polyoxyalkylénée de type (AB)n est constituée d'unités répétitives de formule générale suivante :

$$[SiMe_2\text{-O-}(SiMe_2O)_xSiMe_2\text{ - R-N(H)-R'-O -}(C_2H_4O)_a\text{ - }(C_3H_6O)_b\text{ - R'-N(H)-R-}] \quad (I)$$

dans laquelle :

- a est un nombre entier supérieur ou égal à 1, de préférence compris entre 5 et 200 et encore plus particulièrement entre 5 et 100.
- b est un nombre entier compris entre 0 et 200, de préférence compris entre 4 et 200 et encore plus particulièrement entre 5 et 100.
- R, identiques ou différents, représentent un groupe organique divalent qui est lié à l'atome de silicium adjacent par une liaison carbone-silicium et à un atome d'azote,
- R', identiques ou différents, représentent un groupe organique divalent qui est lié à l'atome d'oxygène adjacent par une liaison carbone-oxygène et à un atome d'azote,

3. Composition selon la revendication 2, **caractérisée par le fait que** lesdits radicaux R et R' sont des radicaux hydrocarbonés en $C_2$-$C_{12}$ comportant éventuellement un ou plusieurs hétéroatomes tels que l'oxygène.

4. Composition selon la revendication 3, **caractérisée par le fait que** ledit radical R désigne un radical éthylène, un radical propylène linéaire ou ramifié, un radical butylène linéaire ou ramifié ou un radical $-CH_2CH_2CH_2OCH(OH)CH_2-$.

5. Composition selon la revendication 3, **caractérisée par le fait que** le radical R' désigne un radical alkylène divalent comme par exemple l'éthylène, le propylène linéaire ou ramifié ou le butylène linéaire ou ramifié.

6. Composition selon l'une quelconque des revendications précédentes, **caractérisée par le fait que** les poly-$\alpha$-oléfines sont de type polybuténe, hydrogéné ou non, ou de type polydécène, hydrogéné ou non.

7. Composition selon l'une quelconque des revendications 1 à 5, **caractérisée par le fait que** les esters d'acides sont choisis parmi le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; le myristate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle ; le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle .

8. Composition selon l'une quelconque des revendications 1 à 5, **caractérisée par le fait que** les huiles fluorées, les cires fluorées, les gommes fluorées sont choisies parmi les perfluoropolyéthers et les composés fluorohydrocarbonées.

9. Composition selon l'une quelconque des revendications 1 à5, **caractérisée par le fait que** ledit polymère cationique est choisi parmi les dérivés d'éther de cellulose quaternaires, les cyclopolymères, les polysaccharides cationiques et leurs mélanges.

10. Composition selon la revendication 9, **caractérisée par le fait que** ledit cyclopolymère est choisi parmi les homopolymères du chlorure de diallyldiméthylammonium et les copolymères du chlorure de diallyldiméthylammonium et d'acrylamide.

**11.** Composition selon la revendication 9, **caractérisée par le fait que** lesdits dérivés d'éther de cellulose quaternaires sont choisis parmi les hydroxyéthylcelluloses ayant réagi avec un époxyde substitué par un groupement triméthylammonium.

**12.** Composition selon la revendication 9, **caractérisée par le fait que** lesdits polysaccharides cationiques sont choisis parmi les gommes de guar modifiées par un sel de 2,3-époxypropyl triméthylammonium.

**13.** Composition selon l'une quelconque des revendications 1 à 5, **caractérisée par le fait que** les silicones sont choisies parmi les polyorganosiloxanes insolubles dans la composition et se présentent sous forme d'huiles, de cires, de résines ou de gommes.

**14.** Composition selon la revendication 13, **caractérisée par le fait que** les polyorganosiloxanes sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

**15.** Composition selon la revendication 14, **caractérisée par le fait que** :

(a) les polyalkylsiloxanes sont choisis parmi :

- les polydiméthylsiloxanes à groupements terminaux triméthylsilyle ;
- les polydiméthylsiloxanes à groupements terminaux diméthylsilanol ;
- les polyalkyl($C_1$-$C_{20}$)siloxanes ;

(b) les polyalkylarylsiloxanes sont choisis parmi :

- les polydiméthylméthylphénylsiloxanes, les polydiméthyldiphényl siloxanes linéaires et/ou ramifiés de viscosité comprise entre $1.10^{-5}$ et $5.10^{-2} m^2/s$ à 25°C ;

(c) les gommes de silicone sont choisies parmi les polydiorganosiloxanes ayant des masses moléculaires moyennes en nombre comprises entre 200 000 et 1 000 000 utilisés seuls ou sous forme de mélange dans un solvant ;

(d) les résines sont choisies parmi les résines constituées d'unités : $R_3\ Si\ O_{1/2}$, $R_2\ Si\ O_{2/2}$, $R\ Si\ O_{3/2}$, $Si\ O_{4/2}$ dans lesquelles R représente un groupement hydrocarboné ayant de 1 à 16 atomes de carbone ou un groupement phényle ;

(e) les silicones organo modifiées sont choisies parmi les silicones comportant dans leur structure un ou plusieurs groupements organofonctionnels fixés par l'intermédiaire d'un radical hydrocarboné.

**16.** Composition selon la revendication 15, **caractérisée par le fait que** les gommes de silicone utilisées seules ou sous forme de mélange sont choisies parmi les structures suivantes :

- polydiméthylsiloxane
- polydiméthylsiloxane/méthylvinylsiloxanes,
- polydiméthylsiloxane/diphénylsiloxane,
- polydiméthylsiloxane/phénylméthylsiloxane,
- polydiméthylsiloxane/diphénylsiloxane/méthylvinylsiloxanes et les mélanges suivants :
  - des mélanges formés à partir d'un polydiméthylsiloxane hydroxylé en bout de chaîne et d'un polydiméthylsiloxane cyclique ;
  - les mélanges formés à partir d'une gomme polydiméthylsifoxane et d'une silicone cyclique ; et
  - des mélanges de polydiméthylsiloxanes de viscosités différentes.

**17.** Composition selon la revendication 15, **caractérisée par le fait que** les silicones organomodifiées sont choisies parmi les polyorganosiloxanes comportant :

a) des groupements polyéthylèneoxy et/ou polypropylèneoxy ;

b) des groupements aminés substitués ou non ;
c) des groupements thiols ;
d) des groupements alcoxylés ;
e) des groupements hydroxyalkyle répondant à la formule suivante :

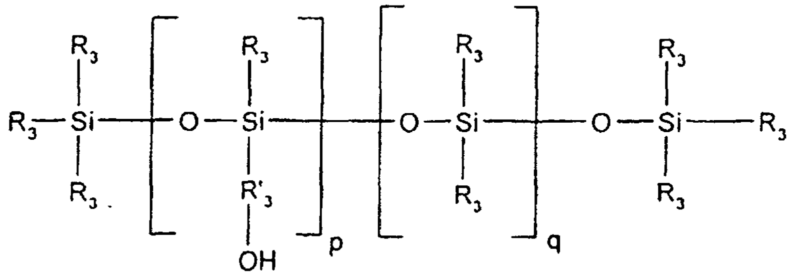

(V)

dans laquelle les radicaux $R_3$ identiques ou différents sont choisis parmi les radicaux méthyle et phényle, au moins 60 % en mole des radicaux $R_3$ représentant méthyle ; le radical $R'_3$ est un chaînon alkylène divalent hydrocarboné en $C_2$-$C_{18}$ ; p est compris entre 1 et 30 inclus ; q est compris entre 1 et 150 inclus ;
f) des groupements acyloxyalkyle répondant à la formule suivante :

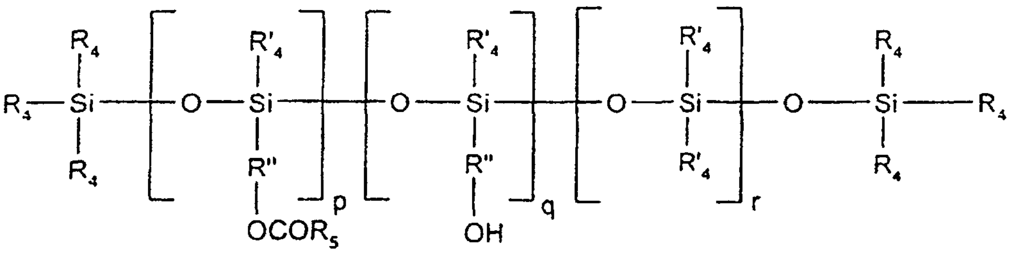

(VI)

dans laquelle :

$R_4$ désigne méthyle, phényle, -$OCOR_5$, hydroxyle, un seul des radicaux $R_4$ par atome de silicium pouvant être OH ;
$R'_4$ désigne méthyle, phényle ; au moins 60 % en mole de l'ensemble des radicaux $R_4$ et $R'_4$ désignant méthyle ;
$R_5$ désigne alkyle ou alcényle en $C_8$-$C_{20}$ ;
R'' désigne un radical alkylène hydrocarboné divalent, linéaire ou ramifié en $C_2$-$C_{18}$ ;
r est compris entre 1 et 120 inclus ;
p est compris entre 1 et 30 ;
q est égal à 0 ou est inférieur à 0,5 p, p + q étant compris entre 1 et 30 ; les polyorganosiloxanes de formule (VI) pouvant contenir des groupements ;

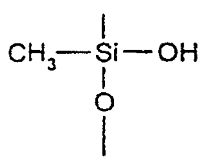

dans des proportions ne dépassant pas 15 % de la somme p + q + r ;

g) des groupements alkyl carboxyliques,
h) des groupements 2-hydroxyalkylsulfonates,
i) des groupements 2-hydroxyalkylthiosulfonates,
j) des groupements hydroxyacylamino.

18. Composition selon l'une quelconque des revendications 13 à 17, **caractérisée par le fait que** les polyorganosiloxanes sont choisis parmi les polyalkylsiloxanes à groupements terminaux triméthylsilyle, les polyalkylsiloxanes à groupements terminaux diméthylsilanol, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes.

19. Composition selon l'une quelconque des revendications précédentes, **caractérisée par le fait qu'**elle comprend en outre au moins un agent tensioactif choisi parmi les tensioactifs anioniques, cationiques, non ioniques, amphotères et leurs mélanges.

20. Composition selon la revendication 19, **caractérisée par le fait que** le ou les agents tensioactifs sont présents à une concentration comprise entre 0,1% et 40% en poids, de préférence entre 3% et 40% en poids, et encore plus préférentiellement entre 5% et 30% en poids, par rapport au poids total de la composition.

21. Composition selon l'une quelconque des revendications précédentes, **caractérisée en ce que** l'agent conditionneur est présent à une concentration comprise entre 0,001 % et 10 % en poids par rapport au poids total de la composition, de préférence entre 0,005 % et 7 % en poids et en particulier entre 0,01 % et 5 % en poids.

22. Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

23. Utilisation d'une composition telle que définie dans l'une quelconque des revendications précédentes pour le lavage ou pour le soin des matières kératiniques.

24. Procédé de traitement des matières kératiniques, telles que les cheveux, **caractérisé en ce qu'**il consiste à appliquer sur lesdites matières une composition cosmétique selon l'une des revendications 1 à 22.

## Patentansprüche

1. Kosmetische Zusammensetzung, **dadurch gekennzeichnet, dass** sie in einem kosmetisch akzeptablen Medium mindestens ein aminiertes polyalkoxyliertes Silicon vom Typ $(AB)_n$, worin A einen Polysiloxanblock und B einen polyalkoxylierten Block bedeutet, der mindestens eine Aminogruppe enthält, und mindestens ein Konditioniermittel enthält, das unter den Poly-$\alpha$-olefinen, fluorierten Ölen, fluorierten Wachsen, fluorierten Gummis, Carbonsäureestern mit mindestens 10 Kohlenstoffatomen, kationischen Polymeren, in dem Medium unlöslichen Siliconen, Mineralölen, pflanzlichen Ölen und tierischen Ölen ausgewählt ist.

2. Zusammensetzung nach Anspruch 1, **dadurch gekennzeichnet, dass** das aminierte polyalkoxylierte Silicon vom Typ (AB)n aus wiederkehrenden Einheiten der folgenden allgemeinen Formel besteht:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-} R'\text{-}N(H)\text{-}R\text{-}] \quad (I),$$

worin bedeuten:

- a 1 oder eine ganze Zahl über 1, vorzugsweise im Bereich von 5 bis 200 und insbesondere im Bereich von 5 bis 100;
- b 0 oder eine ganze Zahl im Bereich von 1 bis 200, vorzugsweise im Bereich von 4 bis 200 und insbesondere im Bereich von 5 bis 100;
- die Gruppen R, die gleich oder verschieden sind, eine zweiwertige organische Gruppe, die über eine Kohlenstoff-Silicium-Bindung an das angrenzende Siliciumatom und an ein Stickstoffatom gebunden ist; und
- die Gruppen R', die gleich oder verschieden sind, eine zweiwertige organische Gruppe, die über eine Kohlenstoff-Sauerstoff-Bindung an das angrenzende Sauerstoffatom und an ein Stickstoffatom gebunden ist.

3. Zusammensetzung nach Anspruch 2, **dadurch gekennzeichnet, dass** die Gruppen R und R' $C_{2\text{-}12}$-Kohlenwas-

serstoffgruppen sind, die gegebenenfalls ein oder mehrere Heteroatome, wie beispielsweise Sauerstoff, enthalten.

4. Zusammensetzung nach Anspruch 3, **dadurch gekennzeichnet, dass** die Gruppe R eine Ethylengruppe, eine geradkettige oder verzeigte Propylengruppe, eine geradkettige oder verzeigte Butylengruppe oder die Gruppe $-CH_2CH_2CH_2OCH(OH)CH_2-$ bedeutet.

5. Zusammensetzung nach Anspruch 3, **dadurch gekennzeichnet, dass** die Gruppe R' eine zweiwertige Alkylengruppe, beispielsweise eine Ethylengruppe, eine geradkettige oder verzeigte Propylengruppe oder eine geradkettige oder verzeigte Butylengruppe bedeutet.

6. Zusammensetzung nach einem der vorhergehenden Ansprüchen, **dadurch gekennzeichnet, dass** die Poly-$\alpha$-olefine vom hydrierten oder nicht hydrierten Polybutentyp oder vom hydrierten oder nicht hydrierten Polydecentyp sind.

7. Zusammensetzung nach einem der Ansprüche 1 bis 5, **dadurch gekennzeichnet, dass** die Carbonsäureester unter Dihydroabietylbehenat; Octyldodecylbehenat; Isocetylbehenat; Cetyllactat; $C_{12-15}$-Alkyllactat; Isostearyllactat; Lauryllactat; Linoleyllactat; Oleyllactat; (Iso)stearyloctanoat; Isocetyloctanoat; Octyloctanoat; Cetyloctanoat; Decyloleat; Isocetylisostearat; Isocetyllaurat; Isocetylstearat; Isodecyloctanoat; Isodecyloleat; Isononylisononanoat; Isostearylpalmitat; Methylacetylricinoleat; Myristylstearat; Octylisononanoat; 2-Ethylhexylisononat; Octylpalmitat; Octylperlagonat; Octylstearat; Octyldodecylerucat; Octyldodecylmyristat; Oleylerucat; Ethylpalmitat; Isopropylpalmitat; 2-Ethylhexylpalmitat; 2-Octyldecylpalmitat; Alkylmyristaten, wie Isopropylmyristat, Butylmyristat, Cetylmyristat und 2-Octyldodecylmyristat; Hexylstearat; Butylstearat; Isobutylstearat; Dioctylmalat; Hexyllaurat; 2-Hexyldecyllaurat; Diethylsebacat; Diisopropylsebacat; Düsopropyladipat; Di-n-propyladipat; Dioctyladipat; Diisostearyladipat; Dioctylmaleat; Glycerylundecylenat; Octyldodecylstearoylstearat; Pentaerythritylmonoricinoleat; Pentaerythrityltetraisononanoat; Pentaerythrityltetrapelargonat; Pentaerythrityltetraisostearat; Pentaerythrityltetraoctanoat; Propylenglykoldicaprylat; Tridecylerucat; Triisopropylcitrat; Triisostearylcitrat; Glyceryltrilactat; Glyceryltrioctanoat; Trioctyldodecylcitrat; und Trioleylcitrat ausgewählt sind.

8. Zusammensetzung nach einem der Ansprüche 1 bis 5, **dadurch gekennzeichnet, dass** die fluorierten Öle, fluorierten Wachse und fluorierten Gummis unter den Perfluorpolyethern und Fluorkohlenwasserstoffen ausgewählt sind.

9. Zusammensetzung nach einem der Ansprüche 1 bis 5, **dadurch gekennzeichnet, dass** das kationische Polymer unter den quartären Cellulosetherderivaten, Cyclopolymeren, kationischen Polysacchariden und deren Gemischen ausgewählt ist.

10. Zusammensetzung nach Anspruch 9, **dadurch gekennzeichnet, dass** das Cyclopolymer unter den Diallyldimethylammoniumchlorid-Homopolymeren un den Dimethyldiallylammoniumchlorid/Acrylamid-Copolymeren ausgewählt ist.

11. Zusammensetzung nach Anspruch 9, **dadurch gekennzeichnet, dass** die quartären Cellulosetherderivate unter den Hydroxyethylcellulosen ausgewählt sind, die mit einem mit einer Trimethylammoniumgruppe substituierten Epoxid umgesetzt wurden.

12. Zusammensetzung nach Anspruch 9, **dadurch gekennzeichnet, dass** die kationischen Polysaccharide unter den mit einem 2,3-Epoxypropyltrimethylammoniumsalz modifizierten Guargummen ausgewählt sind.

13. Zusammensetzung nach einem der Ansprüche 1 bis 5, **dadurch gekennzeichnet, dass** die Silicone unter den in der Zusammensetzung unlöslichen Polyorganosiloxanen ausgewählt sind und in Form von Ölen, Wachsen, Harzen oder Gummis vorliegen.

14. Zusammensetzung nach Anspruch 13, **dadurch gekennzeichnet, dass** die Polyorganosiloxane nicht flüchtige Polyorganosiloxane sind, die unter den Polyalkylsiloxanen, Polyarylsiloxanen, Polyalkylarylsiloxanen, Silicongummis, Siliconharzen, mit organofunktionellen Gruppen modifizierten Polyorganosiloxanen sowie deren Gemischen ausgewählt sind.

15. Zusammensetzung nach Anspruch 14, **dadurch gekennzeichnet, dass**

(a) die Polyalkylsiloxane unter den folgenden Verbindungen ausgewählt sind:

- den Polydimethylsiloxanen mit endständigen Trimethylsilylgruppen;
- den Polydimethylsiloxanen mit endständigen Dimethylsilanolgruppen;
- den Poly($C_{1-20}$)alkylsiloxanen;

(b) die Polyalkylarylsiloxane unter den folgenden Verbindungen ausgewählt sind:

- den Polydimethylmethylphenylsiloxanen und Polydimethyldiphenylsiloxanen, die geradkettig und/oder verzweigt vorliegen und bei 25°C eine Viskosität von $1 \cdot 10^{-5}$ bis $5 \cdot 10^{-2}$ $m^2/s$ aufweisen;

(c) die Silicongummis unter den Polydiorganosiloxanen mit einem Zahlenmittel des Molekulargewichts im Bereich von 200 000 bis 1 000 000, die als solche oder im Gemisch mit einem Lösungsmittel verwendet werden, ausgewählt sind;
(d) die Harze unter den Harzen ausgewählt sind, die aus Einheiten: $R_3SiO_{1/2}$, $R_2SiO_{2/2}$, $RSiO_{3/2}$ und $SiO_{4/2}$ bestehen, wobei die Gruppe R eine Kohlenwasserstoffgruppe mit 1 bis 16 Kohlenstoffatomen oder eine Phenylgruppe bedeutet;
(e) die organomodifizierten Silicone unter den Siliconen ausgewählt sind, die in ihrer Struktur eine oder mehrere organofunktionelle Gruppen enthalten, die über eine Kohlenwasserstoffgruppe gebunden sind.

**16.** Zusammensetzung nach Anspruch 15, **dadurch gekennzeichnet, dass** die Silicongummis, die einzeln oder in Form von Gemischen verwendet werden, unter den folgenden Strukturen:

- Polydimethylsiloxan,
- Polydimethylsiloxan/methylvinylsiloxanen,
- Polydimethylsiloxan/diphenylmethylsiloxan,
- Polydimethylsiloxan/phenylmethylsiloxan,
- Polydimethylsiloxan/diphenylsiloxan/methylvinylsiloxanen; und

den folgenden Gemischen ausgewählt sind:

- Gemischen, die aus einem am Kettenende hydroxylierten Polydimethylsiloxan und einem cyclischen Polydimethylsiloxan gebildet werden,
- Gemischen, die aus einem Polydimethylsiloxangummi und einem cyclischen Silicon gebildet werden, und
- Gemischen von zwei Polydimethylsiloxanen unterschiedlicher Viskositäten.

**17.** Zusammensetzung nach Anspruch 15, **dadurch gekennzeichnet, dass** die organomodifizierten Silicone unter den Polyorganosiloxanen ausgewählt sind, die enthalten:

a) Polyethylenoxy- und/oder Polypropylenoxygruppen,
b) substituierte oder unsubstituierte aminierte Gruppen,
c) Thiolgruppen,
d) alkoxylierte Gruppen,
e) Hydroxylalkylgruppen, die der folgenden Formel entsprechen:

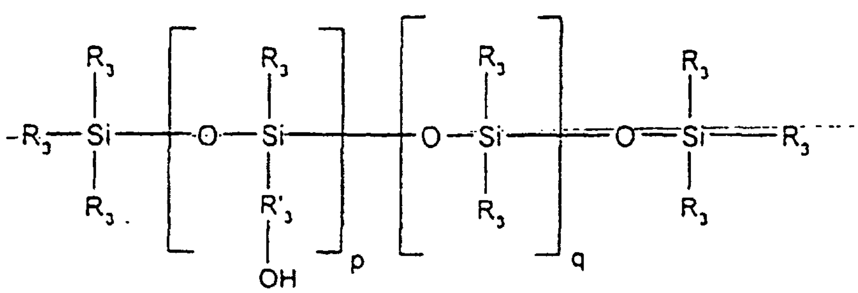

(V)

wobei die Gruppen $R_3$, die identisch oder voneinander verschieden sind, unter Methyl oder Phenyl ausgewählt sind, wobei mindestens 60 Mol-% der Gruppen $R_3$ Methyl bedeuten; die Gruppe $R'_3$ ist eine zweiwertige Alkylenkette mit 2 bis 18 Kohlenstoffatomen; p liegt im Bereich von 1 bis 30; und q liegt im Bereich von 1 bis 150;

f) Acyloxyalkylgruppen, die der folgenden Formel entsprechen:

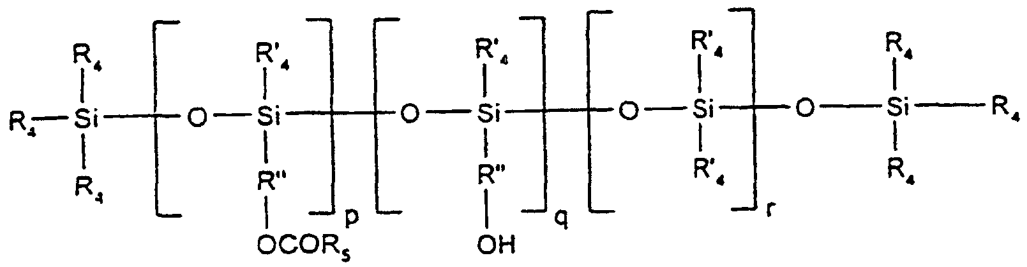

(VI)

worin bedeuten:

$R_4$ Methyl, Phenyl, $-OCOR_5$, Hydroxy, wobei eine Gruppe $R_4$ pro Siliciumatom OH bedeuten kann;
$R'_4$ Methyl oder Phenyl: mindestens 60 Mol-% der Gruppen $R_4$ und $R'_4$ bedeuten Methyl;
$R_5$ Alkyl oder Alkenyl mit 8 bis 20 Kohlenstoffatomen;
R" eine geradkettige oder verzweigte zweiwertige Alkylengruppe mit 2 bis 18 Kohlenstoffatomen;
r liegt im Bereich von 1 bis 120;
p liegt im Bereich von 1 bis 30;
q bedeutet 0 oder liegt unter 0,5 p, wobei p + q im Bereich von 1 bis 30 liegt; die Polyorganosiloxane der Formel (VI) können Gruppen:

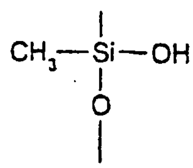

in Mengenanteilen enthalten, die 15 % der Summe p + q + r nicht übersteigen;

g) Alkylcarboxygruppen,
h) 2-Hydroxyalkylsulfonatgruppen,
i) 2-Hydroxyalkylthiosulfonatgruppen, und
j) Hydroxyacylaminogruppen.

**18.** Zusammensetzung nach einem der Ansprüche 13 bis 17, **dadurch gekennzeichnet, dass** die Polyorganosiloxane unter den Polyalkylsiloxanen mit Trimethylsilylendgruppen, den Polyalkylsiloxanen mit Dimethylsilanolendgruppen, den Polyalkylarylsiloxanen, den Gemischen von zwei PDMS, die aus einem Gummi und einem Öl mit unterschiedlichen Viskositäten bestehen, den Gemischen von Organosiloxanen und cyclischen Siliconen und den Organopolysiloxanharzen ausgewählt sind.

**19.** Zusammensetzung nach einem der vorhergehenden Ansprüche, **dadurch gekennzeichnet, dass** sie ferner mindestens einen grenzflächenaktiven Stoff enthält, der unter den anionischen, kationischen, nichtionischen oder amphoteren grenzflächenaktiven Stoffen oder deren Gemischen ausgewählt ist.

**20.** Zusammensetzung nach Anspruch 19, **dadurch gekennzeichnet, dass** der grenzflächenaktive Stoff oder die grenzflächenaktiven Stoffe in einer Menge von 0,1 bis 40 Gew.-%, vorzugsweise 3 bis 40 Gew.-% und noch bevorzugter 5 bis 30 Gew.-%, bezogen auf das Gesamtgewicht der Zusammensetzung, vorliegen.

**21.** Zusammensetzung nach einem der vorhergehenden Ansprüche, **dadurch gekennzeichnet, dass** das Konditioniermittel in einer Konzentration von 0,001 bis 10 Gew.-%, vorzugsweise 0,005 bis 7 Gew.-% und insbesondere 0,01 bis 5 Gew.-%, bezogen auf das Gesamtgewicht der Zusammensetzung, vorliegt.

**22.** Zusammensetzung nach einem der vorhergehenden Ansprüche, **dadurch gekennzeichnet, dass** sie als Haarwaschmittel, Haarpflegemittel, Zusammensetzung für eine Dauerwelle, Entkräuselung, Färbung oder Entfärbung der Haare, Zusammensetzung, die ausgespült wird und zwischen den beiden Schritten einer Dauerwelle oder Entkräuselung aufgetragen wird, oder reinige Zusammensetzung für den Körper vorliegt.

**23.** Verwendung einer Zusammensetzung nach einem der vorhergehenden Ansprüche zum Waschen oder zur Pflege von Keratinsubstanzen.

**24.** Verfahren zur Behandlung von Keratinsubstanzen, wie den Haaren, **dadurch gekennzeichnet, dass** es darin besteht, auf die Keratinsubstanzen eine kosmetische Zusammensetzung nach einem der Ansprüche 1 bis 22 aufzubringen.

## Claims

**1.** Cosmetic composition, **characterized in that** it comprises, in a cosmetically acceptable medium, at least one polyoxyalkylenated aminosilicone of (AB)n type, A being a polysiloxane block and B being a polyoxyalkylenated block containing at least one amine group and at least one conditioner chosen from poly-$\alpha$-olefins, fluoro oils, fluoro waxes, fluoro gums, carboxylic acid esters having at lease 10 carbon atoms, cationic polymers, silicones that are insoluble in the medium, and mineral, plant or animal oils.

**2.** Composition according to Claim 1, **characterized in that** the polyoxyalkylenated aminosilicone of (AB)n type is composed of repeating units of the following general formula:

$$[SiMe_2\text{-}O\text{-}(SiMe_2O)_xSiMe_2\text{-}R\text{-}N(H)\text{-}R'\text{-}O\text{-}(C_2H_4O)_3\text{-}(C_3H_6O)_b\text{-}R'\text{-}N(H)\text{-}R\text{-}J \qquad (I)$$

in which:

- a is an integer greater than or equal to 1, preferably between 5 and 200 and even more particularly between 5 and 100,
- b is an integer between 0 and 200, preferably between 4 and 200 and even more particularly between 5 and 100,
- R, which may be identical or different, represent a divalent organic group which is linked to the adjacent silicon atom via a carbon-silicon bond and to a nitrogen atom,
- R', which may be identical or different, represent a divalent organic group which is linked to the adjacent oxygen atom via a carbon-oxygen bond and to a nitrogen atom.

**3.** Composition according to Claim 2, **characterized in that** the said radicals R and R' are $C_2$-$C_{12}$ hydrocarbon-based radicals optionally containing one or more hetero atoms such as oxygen.

**4.** Composition according to Claim 3, **characterized in that** the said radical R denotes an ethylene radical, a linear or branched propylene radical, a linear or branched butylene radical or a $-CH_2CH_2CH_2OCH(OH)CH_2-$ radical.

**5.** Composition according to Claim 3, **characterized in that** the radical R' denotes a divalent alkylene radical such as, for example, ethylene, linear or branched propylene or linear or branched butylene.

**6.** Composition according to any one of the preceding claims, **characterized in that** the poly-$\alpha$-olefine are of hydrogenated or non-hydrogenated polybutene type or of hydrogenated or non-hydrogenated polydecene type.

**7.** Composition according to any one of Claims 1 to 5, **characterised in that** the acid esters are chosen from dihydroabietyl behenate; octyldodecyl behenate; isocetyl behenate; cetyl lactate; $C_{12}$-$C_{15}$ alkyl lactate; isostearyl lactate; lauryl lactate; linoleyl lactate; oleyl lactate; (iso)stearyl octanoate; isocetyl octanoate; octyl octanoate; cetyl octanoate; decyl oleate; isocetyl isostearate; isocetyl laurate; isocetyl stearate; isodecyl octanoate; isodecyl oleate; isononyl isononanoate; isostearyl palmitate; methylacetyl ricinoleate; myristyl stearate; octyl isononanoate; 2-ethylhexyl isononate; octyl palmitate; octyl pelargonate; octyl stearate; octyldodecyl erucate; octyldodecyl myristate; oleyl erucate; ethyl and isopropyl palmitates, 2-ethylhexyl palmitate, 2-octyldecyl palmitate, alkyl myristates such as isopropyl, butyl, cetyl or 2-octyldodecyl myristate, hexyl stearate, butyl stearate, isobutyl stearate; dioctyl malate, hexyl laurels, 2-hexyldecyl laurate; diethyl sebacate; diisopropyl sebacate; diisopropyl adipate; di-n-propyl adipate; dioctyl adipate; diisostearyl adipate; dioctyl maleate; glyceryl undecylenate; octyldodecylstearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate dicaprate; tridecyl erucate; triisopropyl citrate; triisostearyl citrate; glyceryl trilactate; glyceryl trioctanoate; trioctyldodecyl citrate; trioleyl citrate.

**8.** Composition according to any one of Claims 1 to 5, **characterized in that** the fluoro oils, the fluoro waxes and the fluoro gums are chosen from perfluoropolyethers and fluorohydrocarbon compounds.

**9.** Composition according to any one of Claims 1 to 5, **characterized in that** the said cationic polymer is chosen from quaternary cellulose ether derivatives, cyclopolymers and cationic polysaccharides, and the mixtures thereof.

**10.** Composition according to Claim 9, **characterized in that** the said cyclopolymer is chosen from diallyldimethylammonium chloride homopolymers and copolymers of diallyldimethylammonium chloride and of acrylamide.

**11.** Composition according to Claim 9, **characterized in that** the said quaternary cellulose ether derivatives are chosen from hydroxyethylcelluloses which have reacted with an epoxide substituted with a trimethylammonium group.

**12.** Composition according to Claim 9, **characterized in that** the said cationic polysaccharides are chosen from guar gums modified with a 2,3-epoxypropyltrimethylammonium salt.

**13.** Composition according to any one of Claims 1 to 5, **characterized in that** the silicones are chosen from polyorganosiloxanes that are insoluble in the composition and that are in the form of oils, waxes, resins or gums.

**14.** Composition according to Claim 13, **characterized in that** the polyorganosiloxanes are nonvolatile polyorganosiloxanes chosen from polyalkylsiloxanes, polyarylsiloxanes, polyalkylarylsiloxanes, silicone gums and resins, polyorganosiloxanes modified with organofunctional groups, and mixtures thereof.

**15.** Composition according to Claim 14, **characterized in that**:

(a) the polyalkylsiloxanes are chosen from:

- polydimethylsiloxanes containing trimethylsilyl end groups;
- polydimethylsiloxanes containing dimethylsilanol end groups;
- poly($C_1$-$C_{20}$)alkylsiloxanes;

(b) the polyalkylarylsiloxanes are chosen from:

- polydimethylmethylphenylsiloxanes, linear and/or branched polydimethyldiphenylsiloxanes with a viscosity of between $1\times10^{-5}$ and $5\times10^{-2}$ $m^2/s$ at 25°C;

(c) the silicone gums are chosen from polydiorganosiloxanes having number-average molecular masses of between 200,000 and 1,000,000, used alone or in the form of a mixture in a solvent;
(d) the resine are chosen from resins composed of units: $R_3SiO_{1/2}$, $R_2SiO_{2/2}$, $RSiO_{3/2}$, $SiO_{4/2}$ in which R represents a hydrocarbon-based group having from 1 to 16 carbon atoms or a phenyl group;
(e) the organomodified silicones are chosen from silicones containing, in their structure, one or more organofunctional groups attached via a hydrocarbon-based radical.

**16.** Composition according to Claim 15,
**characterized in that** the silicone gums used, alone or in the form of a mixture, are chosen from the following structures:

- polydimethylsiloxane
- polydimethylsiloxane/methylvingsiloxanes,
- polydimethylsiloxane/diphenylsiloxane,
- polydimethylsiloxane/phenylmethylsiloxane,

- polydimethylsiloxane/diphenylsiloxane/methylvinylsiloxanes and the following mixtures:

  - mixtures formed from a polydimethylsiloxane hydroxylated at the end of the chain and from a cyclic polydimethylsiloxane;
  - mixtures formed from a polydimethylsiloxane gum and from a cyclic silicone; and
  - mixtures of polydimethylsiloxanes of different viscosities.

**17.** Composition according to Claim 15, **characterized in that** the organomodified silicones are chosen from polyorganosiloxanes containing:

a) polyethylenoxy and/or polypropylenoxy groups;
b) substituted or unsubstituted amine groups;
c) thiol groups;
d) alkoxylated groups;
e) hydroxyalkyl groups corresponding to the following formula:

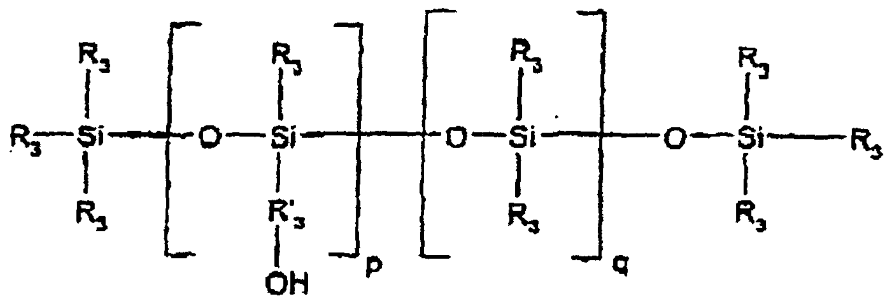

(V)

in which the radicals $R_3$, which may be identical or different, are chosen from methyl and phenyl radicals; at least 60 mol% of the radicals $R_3$ denoting methyl; the radical $R'_3$ is a divalent $C_2$-$C_{18}$ hydrocarbon-based alkylene chain unit; p is between 1 and 30 inclusive; q is between 1 and 150 inclusive;
f) acyloxyalkyl groups corresponding to the following formula;

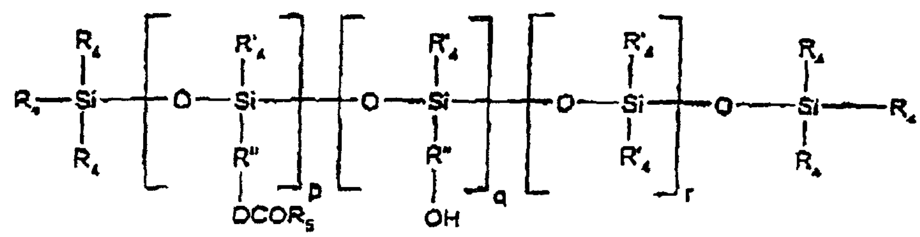

(VI)

in which:

$R_4$ denotes methyl, phenyl, $-OCOR_5$ or hydroxyl, it being possible for only one of the radicals $R_4$ per silicon atom to be OH;
$R'_4$ denotes methyl, phenyl; at least 60 mol% of all of the radicals $R_4$ and $R'_4$, denoting methyl;
$R_5$ denotes $C_8$-$C_{20}$ alkyl or alkenyl;
R" denotes a linear or branched, divalent $C_2$-$C_{18}$ hydrocarbon-based alkylene radical;
r is between 1 and 120 inclusive;
p is between 1 and 30;
q is equal to 0 or is less than 0.5 p, p + q being between 1 and 30; the polyorganosiloxanes of formula (VI) can contain groups:

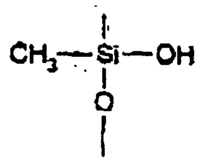

in proportions not exceeding 15% of the sum p + q + r;

g) alkylcarboxylic groups,
h) 2-hydroxyalkyl sulphonate groups,
i) 2-hydroxyalkyl thiosulphonate groups,
j) hydroxyacylamino groups.

**18.** Composition according to any one of Claims 13 to 17, **characterized in that** the polyorganosiloxanes are chosen from polyalkylsiloxanes containing trimethylsilyl end groups, polyalkylsiloxanes containing dimethylsilanol end groups, polyalkylarylsiloxanes, mixtures of two PDMSs consisting of a gum and of an oil of different viscosities, mixtures of organosiloxanes and of cyclic silicones, and organopolysiloxane resins.

**19.** Composition according to any one of the preceding claims, **characterized in that** it also comprises at least one surfactant chosen from anionic, cationic, nonionic and amphoteric surfactants, and mixtures thereof.

**20.** Composition according to Claim 19, **characterized in that** the surfactant(s) is (are) present at a concentration of between 0.1% and 40% by weight, preferably between 3% and 40% by weight and even more preferably between 5% and 30% by weight, relative to the total weight of the composition.

**21.** Composition according to any one of the preceding claims, **characterized in that** the conditioner is present at a concentration of between 0.001% and 10% by weight relative to the total weight of the composition, preferably between 0,005% and 7% by weight and in particular between 0.01% and 5% by weight.

**22.** Composition according to any one of the preceding claims, **characterized in that** it is in the form of a shampoo, a conditioner, a composition for permanent-waving, straightening, dyeing or bleaching the hair, a rinse composition to be applied between the two steps of a permanent-waving or hair-straightening operation, or a washing composition for the body.

**23.** Use of a composition as defined in any one of the preceding claims, to wash or care for keratinous material.

**24.** Process for treating keratinous material, such as the hair, **characterized in that** it consists in applying a cosmetic composition according to one of Claims 1 to 22 to the said material.